# EUROPEAN PATENT SPECIFICATION

(11) **EP 4 052 734 B1**
(45) Date of publication and mention of the grant of the patent: **24.04.2024**
(21) Application number: 22159793.3
(22) Date of filing: 02.03.2022
(51) Int. Cl.: A61L 27/36, A61L 27/38

(54) **IMMUNOCOMPATIBLE TISSUE SCAFFOLD AND METHODS OF FORMING THE SAME**
IMMUNOKOMPATIBLES GEWEBEGERÜST UND VERFAHREN ZU DESSEN HERSTELLUNG
ÉCHAFAUDAGE DE TISSUS IMMUNOCOMPATIBLE ET SES PROCÉDÉS DE FORMATION

(30) Priority: 02.03.2021 CA 3110969; 17.11.2021 US 202117455275
(43) Date of publication of application: 07.09.2022
(73) Proprietor: Solidified Inc., Edmonton, AB T5J 0K4 (CA)
(72) Inventor: Nagendran, Jeevan, T6G 2E1 Edmonton (CA); Nagendran, Jayan, T6G 2E1 Edmonton (CA); Bozso, Sabin J., T6G 2E1 Edmonton (CA)
(74) Representative: HGF

(56) References cited:
- EP-A1- 3 533 475
- WO-A1-2017/127228
- KR-A- 20160 028 253
- BOZSO SABIN J. ET AL: "Comparing Scaffold Design and Recellularization Techniques for Development of Tissue Engineered Heart Valves", REGENERATIVE ENGINEERING AND TRANSLATIONAL MEDICINE, vol. 7, no. 4, 28 July 2020 (2020-07-28), pages 432-439, XP55944753, ISSN: 2364-4133, DOI: 10.1007/s40883-020-00167-x Retrieved from the Internet: URL:https://link.springer.com/article/10.1 007/s40883-020-00167-x/fulltext.html> [retrieved on 2022-07-20]
- BOZSO S J ET AL: "The Role of Autologous Mesenchymal Stem Cell Recellularization in Rescuing the Xenoreactive Immune Response", JOURNAL OF HEART AND LUNG TRANSPLANTATION, ELSEVIER, AMSTERDAM, NL, vol. 39, no. 4, 30 March 2020 (2020-03-30) , XP086111927, ISSN: 1053-2498, DOI: 10.1016/J.HEALUN.2020.01.435 [retrieved on 2020-03-30]
- JINHAE NAM ET AL: "Changes of the Structural and Biomechanical Properties of the Bovine Pericardium after the Removal of ?-Gal Epitopes by Decellularization and ?-Galactosidase Treatment", THE KOREAN JOURNAL OF THORACIC AND CARDIOVASCULAR SURGERY, vol. 45, no. 6, 31 December 2012 (2012-12-31), pages 380-389, XP55755708, ISSN: 2233-601X, DOI: 10.5090/kjtcs.2012.45.6.380

## Description

### TECHNICAL FIELD

This relates to methods of decellularizing xenogenic tissue and methods of forming an immunocompatible scaffold, and in particular, to tissue engineered bioprosthetic heart valve replacements.

### BACKGROUND

Valvular heart disease (VHD) is a relatively common condition with an estimated 2.5% of the world population, and up to 13.3% of those over the age of 75, having VHD. Approximately 250,000 heart valve replacements are performed worldwide every year and the prevalence of VHD is increasing. The number of valve replacements is expected to reach 850,000 per year by 2050. Aortic valve stenosis and mitral valve regurgitation account for up to 75% of current valve disease cases. Historically, rheumatic heart disease due to prior streptococcus pyogenes infection was responsible for the majority of VHD. With the utilization of antibiotic therapy, this cause of VHD has significantly declined in the developed world and has been replaced by degenerative etiologies. Risk factors for the development of VHD include age, male gender, hypertension, smoking, dyslipidemia, coronary artery disease, among others. Growing evidence suggests that COVID-19 can cause heart damage, which could result in increasing incidents of VHD.

There are currently no medical therapies effective in treating VHD. The only definitive management for severe, symptomatic VHD is surgical repair or replacement of the diseased valve. As surgical technique and technologies have improved, valve repair surgery has become increasingly common, although valves that are not amenable to repair require a replacement. There are 3 types of heart valve replacements used today including bioprosthetic or tissue valves, mechanical valves, and homograft valves. Bioprosthetic valves are most commonly composed of bovine pericardial tissue or porcine heart valves. The benefits of bioprosthetic valves include characteristics similar to the native heart valve, lower rates of bleeding when compared to mechanical valves, and the ability to be implanted through a minimally invasive transcatheter aortic valve replacement (TAVR). The main limitation of bioprosthetic valves is their durability. Bioprosthetic valves degrade over time due to a xenogenic immune reaction from the recipient of the valve. Failures rates vary, occurring on average 10-20 years later for elderly patients, but can be significantly shorter for younger patients. In contrast, mechanical valves are more durable than bioprosthetic valves but are highly thrombogenic requiring lifelong anticoagulation to prevent thromboembolic events which carry an increased risk of adverse bleeding events. None of the current options are ideal, and each of the shortfalls is significant. Reoperations and complications due to anticoagulation have a significant impact on the quality of life for patients with VHD as well as adding a significant financial burden to healthcare systems around the world. Bioprosthetic valves are made from bovine or porcine tissue and are susceptible to structural valvular deterioration over time due to an inflammatory immune response from the recipient of the valves. Failure rates vary but can be as high as 50% at 5 years in certain populations with this being especially true for younger patients. Homografts are valves collected from cadaveric donors. These valves are generally associated with improved durability over xenogenic valves (valves made from animal tissue), although these valves are very rare being less common than a heart transplant. Due to their limited availability, homografts are not a feasible option to address the current and future demands for heart valve replacements. EP 3533475 A1 discloses an *ex vivo* method for producing decellularized pericadial material and a recellularized pericardial tissue.

### SUMMARY

In one embodiment there is disclosed a method of forming an immunocompatible scaffold for a recipient. A decellularized tissue matrix is contacted in vitro with eukaryotic cells immunocompatible to the recipient to cover an exterior surface of the decellularized tissue matrix to form the immunocompatible scaffold. The decellularized tissue is formed from tissue xenogenic to the recipient. The decellularized tissue matrix may be contacted with alpha-galactosidase prior to recellularizing the decellularized tissue matrix. This process may be completed in a manner of days, rather than weeks. The immunocompatible scaffold is fixed with a fixing agent such as glutaraldehyde, forming a shelf stable product.

In various embodiments, there may be included any one or more of the following features: the eukaryotic cells further comprise multipotent cells; the multipotent cells further comprise mesenchymal stem cells; the decellularized tissue matrix further comprises a decellularized extracellular matrix; the eukaryotic cells immunocompatible to the recipient further comprise cells homologous or autologous to the recipient; the decellularized tissue matrix is contacted with alpha-galactosidase prior to recellularizing the decellularized tissue matrix; the fixing agent is glutaraldehyde; the decellularized tissue matrix further comprises heart valve tissue; the recipient is human and the tissue is mammalian tissue; the tissue is porcine or bovine tissue; prior to recellularizing the decellularized tissue matrix, obtaining the decellularized tissue matrix by decellularizing tissue xenogenic to the recipient with one or more solutions containing decellularization agents to produce a decellularized tissue matrix and washing the decellularized tissue matrix to remove the decellularization agents; and prior to recellularizing the decellularized tissue matrix, obtaining the decellularized tissue matrix by immersing tissue xenogenic to the recipient in an anionic detergent solution, removing the anionic detergent solution from the tissue, immersing the tissue in a nonionic detergent solution, removing the nonionic detergent solution from the tissue, rinsing the tissue with one or more rinsing solutions to form a decellularized tissue matrix, and contacting the decellularized tissue matrix with alpha-galactosidase.

In various embodiments, there may be included any one or more of the following features: the one or more rinsing solutions collectively comprising a buffer solution, double distilled water, sodium chloride, a nuclease and antibiotics; the decellularized tissue matrix is recellularized by contacting the decellularized tissue matrix with eukaryotic cells immunocompatible to the recipient to form a layer of immunocompatible cells covering the decellularized tissue matrix to form an immunocompatible scaffold; the eukaryotic cells further comprise multipotent cells; the multipotent cells further comprise mesenchymal stem cells; the eukaryotic cells immunocompatible to the recipient further comprise cells homologous or autologous to the recipient; the immunocompatible scaffold is fixed with a fixing agent; the fixing agent is glutaraldehyde; the tissue further comprises heart valve tissue; the recipient is human and the tissue is mammalian tissue; the tissue is porcine or bovine tissue; the immersion in the anionic detergent solution step comprises immersing the tissue in an anionic detergent solution four times, each immersion followed by removing the anionic detergent solution from the tissue; one of the four immersions is carried out with oscillation; the final of the four immersions is between 16 and 24 hours; the immersion in the nonionic detergent solution step comprises immersing the tissue in a nonionic detergent solution five times, each immersion followed by removing the nonionic detergent from the tissue; four of the five immersions are carried out with oscillation; the final of the five immersions is between 16 and 24 hours; the method is completed in less than 4 days; the anionic detergent is sodium dodecyl sulphate; the nonionic detergent is Triton^{™} X-100; the nuclease is a deoxyribonuclease; the tissue comprises fresh tissue, cadaveric tissue, fixed tissue or unfixed tissue; after the rinsing step the decellularized tissue matrix is substantially free of anionic detergent and nonionic detergent such that the decellularized tissue matrix may be readily recellularized; and rinsing the tissue with one or more solutions comprises rinsing the tissue with alternating phosphate-buffered saline and double distilled water, washing the tissue with sodium chloride solution, rinsing the tissue with alternating phosphate-buffered saline and double distilled water, contacting the tissue with a nuclease and magnesium chloride solution, rinsing the tissue with double distilled water, contacting the tissue with a solution of phosphate-buffered saline and antibiotics, and storing the tissue in a buffer and antibiotic solution.

In another embodiment there is disclosed an immunocompatible scaffold comprising a decellularized xenogenic tissue covered with fixed eukaryotic cells immunocompatible to a recipient and forming a shelf-stable product.

In various embodiments there may be included one or more of the following features: the eukaryotic cells cover at least 50% of an external surface of the decellularized tissue matrix; the eukaryotic cells cover at least 80% of an external surface of the decellularized tissue matrix; the eukaryotic cells further comprise multipotent cells; the multipotent cells further comprise mesenchymal stem cells; the eukaryotic cells further comprise cells homologous or autologous to the recipient; the decellularized xenogenic tissue further comprises a decellularized tissue matrix; the decellularized tissue matrix is a native extracellular matrix; the decellularized xenogenic tissue further comprises xenogenic heart valve tissue; the recipient is human and the decellularized xenogenic tissue is decellularized mammalian tissue; the decellularized xenogenic tissue is decellularized porcine or bovine tissue; and the eukaryotic cells form a monolayer on the decellularized xenogenic tissue.

While the disclosed subject-matter will be described in conjunction with the enumerated claims, it will be understood that the exemplified subject-matter is not intended to limit the claims to the disclosed subject-matter. The present invention and the scope thereof is defined by the appended claims. The more generic description of the invention is provided for illustrative purposes only. Embodiments not falling under these claims are for reference purpose only.

### BRIEF DESCRIPTION OF THE FIGURES

Embodiments will now be described with reference to the figures, in which like reference characters denote like elements, by way of example, and in which:
Fig. 1 is a flow chart showing a method of forming an immunocompatible scaffold for a recipient.
Fig. 2 is a flow chart showing a method of decellularizing tissue xenogenic to a recipient.
Fig. 3 is a bar graph showing an enzyme-linked immunosorbent assay (ELISA) analysis of tumor necrosis factor alpha (TNF-α) proinflammatory cytokine expression at day 1 of native, decellularized and recellularized bovine pericardium exposure to human whole blood.
Fig. 4 is a bar graph showing an ELISA analysis of TNF-α proinflammatory cytokine expression at day 3 of native, decellularized and recellularized bovine pericardium exposure to human whole blood.
Fig. 5 is a bar graph showing an interferon gamma (IFN-y) proinflammatory cytokine expression at day 3 of native, decellularized and recellularized bovine pericardium exposure to human whole blood.
Fig. 6 is a graph showing a flow cytometry analysis of native bovine pericardium.
Fig. 7 is a graph showing a proliferation assay under flow cytometry of native bovine pericardium.
Fig. 8 is a graph showing a proliferation assay under flow cytometry of decellularized bovine pericardium.
Fig. 9 is a graph showing a proliferation assay under flow cytometry of human pericardium.
Fig. 10 is a graph showing a flow cytometry analysis of recellularized bovine pericardium.
Fig. 11 is a graph showing a proliferation assay under flow cytometry of recellularized bovine pericardium.
Fig. 12 is a bar graph showing an interleukin 1 beta (IL1β) cytokine concentration at 1 day of native, decellularized and recellularized porcine extracellular matrix exposure to human whole blood.
Fig. 13 is a bar graph showing an IL1β cytokine concentration at 3 days of native, decellularized and recellularized porcine extracellular matrix exposure to human whole blood.
Fig. 14 is a bar graph showing an IL1β cytokine concentration at 5 days of native, decellularized and recellularized porcine extracellular matrix exposure to human whole blood.
Fig. 15 is a bar graph showing a TNF-α cytokine concentration at 1 day of native, decellularized and recellularized porcine extracellular matrix exposure to human whole blood.
Fig. 16 is a bar graph showing a TNF-α cytokine concentration at 3 days of native, decellularized and recellularized porcine extracellular matrix exposure to human whole blood.
Fig. 17 is a bar graph showing a TNF-α cytokine concentration at 5 days of native, decellularized and recellularized porcine extracellular matrix exposure to human whole blood.
Fig. 18 is a hematoxylin and eosin (H&E) and vimentin stain of a decellularized guinea pig valve.
Fig. 19 is a H&E and vimentin stain of a recellularized xenograft.
Fig. 20 is a H&E stain of a syngeneic tissue.
Fig. 21 is a H&E stain of a non-decellularized (fresh) guinea pig xenograft.
Fig. 22 is a H&E stain of a decellularized guinea pig xenograft.
Fig. 23 is a H&E stain of a recellularized guinea pig xenograft.
Fig. 24 is a chart showing total serum immunoglobulin G at 7 days post-implant in a rat model.
Fig. 25 is a graph showing survival and thrombosis of implanted grafts at 7 days post-implant in a rat model.
Fig. 26 is a chart showing a TNF-α cytokine concentration at 1-day post-exposure of native, decellularized and autogenic cell recellularized human pericardium with PBMCs for one day.
Fig. 27 is a chart showing TNF-α cytokine concentration at 1-day post-exposure of native, decellularized and autogenic cell recellularized human pericardium with PBMCs for three days.

### DETAILED DESCRIPTION

In one embodiment there is an initial multi-step, solution-based, decellularization which allows for the removal of surface markers present on xenogenic tissue. Cells immunocompatible with the recipient, such as immunocompatible human mesenchymal stem cells, are used to recellularize the decellularized matrix. This creates a tissue- engineered immunocompatible scaffold, such as a heart valve, that the immune system does not recognize as foreign material, reducing the inflammatory immune response and therefore resulting in reduced deterioration and increased longevity of the immunocompatible scaffold, such as a bioprosthetic heart valve, from the decellularized tissue matrix.

Embodiments of the tissue-engineered heart valves may address certain limitations of current heart valve replacement options. While there are various approaches to tissue engineering, the general idea is to either remove or mask the xenogenic antigens that are thought to elicit the inflammatory immune response from the recipient. This will reduce the rate of degradation over time, increasing the durability of a tissue valve. A tissue valve with increased durability that does not require anticoagulation, is cost-effective, shelf-stable and relatively available would address certain current limitations of valve replacement options.

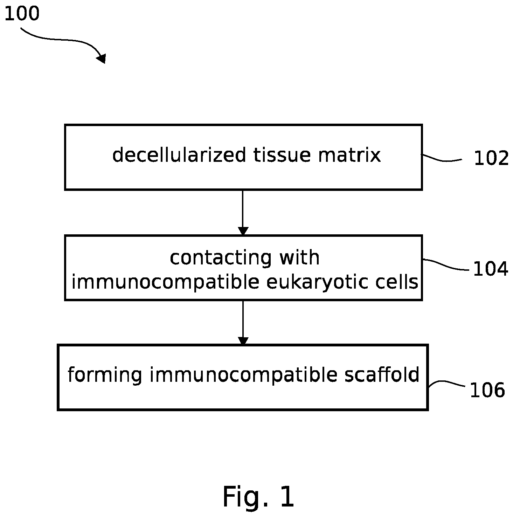

In an embodiment shown in Fig. 1, there is a method 100 of forming an immunocompatible scaffold for a recipient. At 102 the process begins with a decellularized tissue matrix. The matrix is contacted at 104 in vitro with eukaryotic cells immunocompatible to the recipient to adhere to and cover an exterior surface of the decellularized tissue matrix to form at 106 the immunocompatible scaffold.

In one embodiment, reference to an immunocompatible scaffold or immunocompatible cells means a pro-inflammatory cytokine release when exposed to a recipient's blood as expressed as a percentage of the maximal cytokine release for the same amount of the recipient's blood in vitro under the same conditions that is within 10% of the pro-inflammatory cytokine release expected to an autologous tissue or cells to the recipient under the same conditions. Homologous cells and autogenic cells are considered to be immunocompatible cells.

In a preferred embodiment, the tissue may be heart valve tissue. The recipient may be human and the tissue forming the decellularized tissue matrix may be mammalian tissue, such as porcine or bovine tissue. The tissue may be any of fresh tissue, cadaveric tissue, fixed tissue or unfixed tissue. The eukaryotic cells may be any cell capable of repopulating a decellularized tissue matrix, and may be, for example, pluripotent, or multipotent cells. The eukaryotic cells may be endothelial cells. The eukaryotic cells may be mesenchymal stem cells. The eukaryotic cells may be cells allogenic or autologous to the recipient. The eukaryotic cells may be xenogenic cells. The eukaryotic cells may be syngeneic cells. For example, the eukaryotic cells may be primate cells or other close evolutionary neighbours to a human recipient so long as the cells are immunocompatible with the recipient. The eukaryotic cells may be cells that densely adhere to the decellularized tissue matrix. The decellularized tissue matrix may be a decellularized extracellular matrix. The decellularized tissue may be initially formed from any xenogenic tissue, including porcine heart valves and bovine heart valves. The eukaryotic cells, such as mesenchymal stem cells, may be harvested from patients or other sources using techniques known in the art or described here.

The recellularization process described in Fig. 1 may also be used on tissue that is formed from natural or synthetic tissue or on a synthetic matrix or scaffold. The tissue may be xenogenic, allogenic or autologous to the recipient. The tissue may be initially formed from synthetic, engineered or artificial tissue. The tissue matrix may be a natural or synthetic or artificial matrix or scaffold. The decellularized tissue matrix may be contacted with alpha-galactosidase prior to recellularizing the decellularized tissue matrix. The xenogenic scaffold may be fixed with a fixing agent.

Prior to recellularizing the decellularized tissue matrix, the decellularized tissue matrix may be obtained by various methods. For example, tissue xenogenic to the recipient may be decellularized with one or more solutions containing decellularization agents to produce a decellularized tissue matrix. The decellularized tissue matrix may be washed after contact with each of the one or more solutions to remove the decellularization agents and cellular debris. Tissue xenogenic to the recipient has its ordinary meaning.

The decellularized tissue matrix is obtained by immersing tissue xenogenic to the recipient in an anionic detergent solution, removing the anionic detergent solution from the tissue, immersing the tissue in a nonionic detergent solution, removing the nonionic detergent solution from the tissue, rinsing the tissue with one or more rinsing solutions to form a decellularized tissue matrix, and contacting the decellularized tissue matrix with alpha-galactosidase.

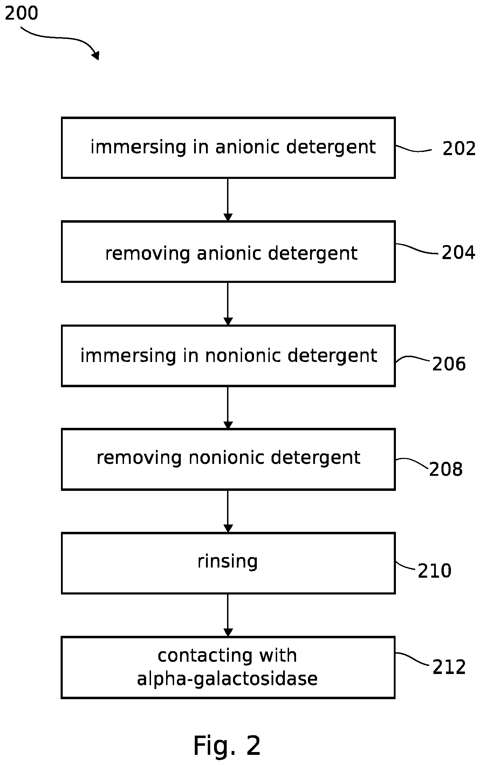

In an embodiment shown in Fig. 2 there is disclosed a method 200 of decellularizing tissue xenogenic to a recipient. For example, the decellularized tissue matrix disclosed in Fig. 1 may be formed using the method disclosed in Fig. 2. At 202, the tissue is immersed in an anionic detergent solution. The anionic detergent may be, for example, sodium dodecyl sulphate (SDS). The SDS may be 0.75%-1.5% SDS or may be 0.75% to 3% SDS. The tissue may be immersed in an anionic detergent solution four times, each immersion followed by removing the anionic detergent solution from the tissue. One of the four immersions may be carried out with oscillation. The final of the four immersions may be between 16 and 24 hours long, and could be, for example, approximately 20 hours. At 204, the anionic detergent solution is removed from the tissue.

At 206, the tissue is immersed in a nonionic detergent solution. The nonionic detergent may be Triton^{™} X-100. The solution may be 0.75% to 1.5% Triton^{™} X-100. The solution may be 0.75% to 3% Triton^{™} X-100. The tissue may be immersed in a nonionic detergent solution five times, each immersion followed by removing the nonionic detergent from the tissue. Four of the five immersions may be carried out with oscillation. The final of the five immersions may be between 16 and 24 hours long and may be, for example, 20 hours. At 208, the nonionic detergent solution is removed from the tissue.

Several detergents and other solutions are known in the art and have been described in decellularization processes previously. These include, but are not limited to, Triton-X 100 (nonionic), Sodium Dodecyl Sulfate (ionic), Trypsin, hypotonic saline solutions, nuclease solutions, hypertonic salt solutions, ethanol, Magnesium Chloride, Calcium Chloride, BRIJ-35, Sodium laureth sulfate, TWEEN 20, LUBROL-PX, and polyethylene lauryl ether. The inventors predict that some or all of these may be effective as additives to the detergent steps described above, or alone as detergents in the decellularization protocol as they have worked previously.

At 210, the tissue is rinsed with one or more rinsing solutions to form a decellularized tissue matrix. The one or more rinsing solutions collectively may include a buffer solution, double distilled water, sodium chloride, a nuclease and antibiotics. The nuclease may be a deoxyribonuclease. Rinsing the tissue may include rinsing the tissue with alternating phosphate-buffered saline (PBS) and double distilled water (ddH20), washing the tissue with sodium chloride solution, rinsing the tissue with alternating phosphate-buffered saline and double distilled water, contacting the tissue with a nuclease and magnesium chloride solution, rinsing the tissue with double distilled water, contacting the tissue with a solution of phosphate-buffered saline and antibiotics, and storing the tissue in a buffer and antibiotic solution. The sodium chloride solution may be 0.5M to 2M sodium chloride or may be 0.5M to 3M sodium chloride. The nuclease and magnesium chloride solution may be 2.5mM to 5.5mM magnesium chloride, or may be 1.5mM to 6.5mM magnesium chloride.

After rinsing, the decellularized tissue matrix may be substantially free of detergents, for example anionic and nonionic detergents, such that the decellularized tissue matrix may be readily recellularized. At 212, the decellularized tissue matrix is contacted with alpha-galactosidase. The contacting with alpha-galactosidase may be accomplished by soaking the decellularized tissue matrix in PBS containing alpha-galactosidase. Once the recellularization process has been completed to form an immunocompatible scaffold, the immunocompatible scaffold is fixed in order to preserve the immunocompatible scaffold and make it shelf stable. The immunocompatible scaffold is fixed using known techniques and fixing agents, including photo-oxidation, thermal fixation, crosslinking fixatives, including aldehydes, and precipitating fixatives, including alcohols. For example, the fixing agent may be glutaraldehyde.

The method 200 may be completed in less than 4 days. Steps 202, 204, 206 and 208 may be completed in less than 3 days.

The methods disclosed herein may be used with any tissue that has an extracellular matrix, including but not limited to vascular tissue, subintestinal submucosa, vessel walls, and mesenchyme derived tissues. Denser tissue may require longer contacting times.

The anionic and nonionic detergents may be any anionic or nonionic detergent known in the art to have decellularization properties. The rinsing solutions may be any rinsing solution or combination of rinsing solutions known to remove cellular debris and detergents from the scaffold.

Following the decellularization process, the decellularized tissue matrix is recellularized by contacting the decellularized tissue matrix with eukaryotic cells immunocompatible to the recipient, under conditions in which the eukaryotic cells adhere to and multiply and/or differentiate within and on the decellularized tissue matrix, to cover the decellularized tissue matrix with immunocompatible cells to form an immunocompatible scaffold, for example, using the process shown in the embodiment in Fig. 1. The decellularized tissue matrix is contacted with the eukaryotic cells for enough time for the eukaryotic cells to adhere to and cover the decellularized tissue matrix and sufficiently mask any xenogenic antigens or detergent remaining on the external surface to produce an immunocompatible scaffold. Cover means that the immunocompatible cells adhered to the decellularized tissue matrix sufficiently mask an external surface of the decellularized tissue matrix to form an immunocompatible scaffold. Masking may be blocking access to the decellularized tissue matrix by components of the immune system such that antigens on the decellularized tissue matrix are not detected by the immune system. Coverage may be partial or complete coverage of the external surface of the decellularized tissue matrix. The eukaryotic cells may cover 50% or more of the external surface of the decellularized tissue matrix. The eukaryotic cells preferably cover 80% or more of the external surface of the decellularized tissue matrix. The immunocompatible scaffold may produce a pro-inflammatory cytokine release when exposed to a recipient's blood as expressed as a percentage of the maximal pro-inflammatory cytokine release for the same amount of the recipient's blood in vitro under the same conditions that is within 10% of the pro-inflammatory cytokine release expected to an autologous tissue to the recipient under the same conditions.

The eukaryotic cells covering the decellularized tissue matrix may form a monolayer or thin layer of cells. The thin layer may be in a range of 1-5 cell layers thick for the initial recellularization. The contacting is done in vitro. The contacting may be done in the presence of a culture medium compatible with the eukaryotic cells and decellularized tissue matrix to aid the recellularization process. The culture medium may include factors to support or enhance the adhesion of the eukaryotic cells to the decellularized tissue matrix, and may include factors to support or enhance the growth and division of the eukaryotic cells. Example of factors include, but are not limited to, addition of trypsin to the decellularized tissue matrix prior to recellularization, addition of VEGF or fibroblast inducible factor 14 to the culture media, linking progenitor cell-specific antibodies (for example, CD34) to the decellularized tissue matrix prior to recellularization, and treatment of decellularized tissue matrix with acetic acid and arginylglycylaspartic acid (RGD) polypeptides.

Following the decellularization process and prior to the recellularization process the decellularized tissue matrix may be immersed in a culture media, for example for 24 hours.

The immunocompatible scaffold is fixed to form a shelf-stable and sterile scaffold which may be stored or shipped before implantation or grafting in a recipient. The shelf stable scaffold may be capable of remaining at room temperature for an extended period of time, such as up to 5 years or longer without degradation. Fixing the immunocompatible scaffold may also decrease the likelihood of the eukaryotic cells sloughing off the decellularized tissue matrix when exposed to aortic pressure after implant into a recipient.

The immunocompatible scaffold may be further exposed to autologous or allogenic blood. The immunocompatible scaffold may be used for in vivo implantation or grafting.

In some embodiments the multi-step decellularization process is effective at removing xenogenic markers from a tissue scaffold. Testing has confirmed the removal of xenogenic markers from a tissue scaffold with bovine pericardium in a human model, porcine extracellular matrix in a human model, guinea pig aortic valve in a rat model. Based on these results, the inventors expect to be able to confirm similar results with porcine aortic leaflet in a human model and human pericardium in a human model.

In some embodiments the decellularized tissue matrix is effective in reducing the inflammatory immune response. Testing has confirmed a reduction of the inflammatory immune response with bovine pericardium in a human model, porcine extracellular matrix in a human model and guinea pig aortic valve in a rat model. Based on these results, the inventors expect to be able to confirm similar results with porcine aortic leaflet in a human model and human pericardium in a human model.

In some embodiments the process of cleaving alpha-galactose is effective in removing the alpha-galactose antigen and is anticipated to further reduce the immune response to the decellularized tissue.

In some embodiments collecting and culturing mesenchymal stem cells from sternal bone marrow has proven effective in producing enough stem cells to recellularize the decellularized tissue matrix.

In some embodiments recellularization has proven effective in recellularizing a decellularized tissue matrix. Testing has confirmed effectiveness in recellularizing a decellularized tissue matrix on bovine pericardium, porcine aortic leaflet, guinea pig aortic valve, intact porcine aortic and pulmonic valves.

In some embodiments the method of recellularization of a decellularized tissue matrix formed from xenogenic tissue is effective in further reducing the inflammatory immune response to the decellularized tissue matrix formed from xenogenic tissue to levels similar to autologous control tissue. Testing has confirmed this effectiveness with bovine pericardium in a human model, porcine extracellular matrix in a human model and guinea pig aortic valve in a rat model. Based on these results, the inventors expect to be able to confirm similar results with porcine aortic leaflet in a human model and human pericardium in a human model.

In some embodiments the methods of decellularization and recellularization reduces the inflammatory immune response to an immunocompatible scaffold to be used for heart valve material which will reduce the rate of structural valvular deterioration increasing the durability of a bioprosthetic heart valve made from this immunocompatible scaffold.

In some embodiments the method has been demonstrated to be effective and applicable for use on porcine heart valve replacements, bovine heart valve replacements, and transcatheter heart valve replacements.

Some embodiments of the method are relatively cost-effective, when compared to other tissue-engineered heart valves, allowing for feasible mass production and utilization by healthcare providers globally.

The inventors anticipate that the cost-effectiveness and benefits of the disclosed tissue-engineered heart valve may improve the quality of life and survival for patients and will reduce long term costs for healthcare systems, when accounting for the reduction in reoperations due to valve degeneration and complications due to anticoagulation use.

Immunocompatible scaffolds of the present invention are shelf-stable after both the decellularization and recellularization processes after which fixation will occur.

Exemplary embodiments of the methods of forming an immunocompatible scaffold as disclosed herein are set out below. In the exemplary embodiments, incubation times set for 1 hour may range from 30 minutes to 2 hours. Incubation times could be outside 30 minutes to 2 hours with modification to concentration of solutions.

### EXAMPLES

### Example 1 - Decellularization

Day 1 - The tissue was placed in a petri dish and washed with enough PBS to cover the tissue and aspirate. Enough SDS was added to completely cover the tissue and the dishes were oscillated at a minimum of 30 oscillations/min for 1 hour at room temperature. The tissue was aspirated to remove SDS. Fresh SDS was added and the SDS wash was repeated two times leaving the last SDS wash overnight.

Day 2 - The SDS solution was aspirated and replaced with enough 1% Triton^{™} X-100 to cover the tissue and was set for a minimum of 1 hr of oscillations at the same setting as previous. This was repeated three times. The Triton^{™} X-100 was aspirated and added again and left overnight.

Day 3 - The tissue was rinsed with 25 mL PBS two times and aspirated after each wash in order to wash away detergent. The tissue was rinsed with 25mL autoclaved double distilled water (ddH2O) eight times in order to wash away any remaining detergent. If there were bubbles at the end of 8 washes indicating residual detergent, the tissue was rinsed until bubbles disappear, then washed two additional times. The liquid was then aspirated. Enough 1M NaCl solution in PBS was added to cover the tissue, and the tissue was placed onto the oscillator at room temperature, at a minimum of 30 RPM for at least 1-Hour. The tissue was then rinsed with enough PBS to cover the tissue and aspirated to remove the PBS. The tissue was then rinsed with enough ddH2O two times to cover the tissue and aspirated to remove the ddH2O. Enough deoxyribonuclease (DNase) + MgCl2 solution was added to cover the tissue and the tissue was placed into a heated shaker at 36-39°C, at a minimum of RPM 25 for at least 1-Hour. The DNase solution was then aspirated. The tissue was then rinsed two times with enough DDH2O to cover the tissue, aspirating after each wash. The tissue was then rinsed with enough PBS and antibiotics solution to cover the tissue in order to remove any bacteria. The tissue was placed onto the oscillator at room temp, RPM 30 for 30-60 min, and the PBS and antibiotics solution were aspirated. A Hanks' Balanced Salt Solution (HBSS) and antibiotics solution was prepared. The tissue was placed into a plastic tube and enough HBSS and antibiotics solution was added. The tissue was then stored at 4°C until the Recellularization step.

At any time after this stage alpha galactose can be optionally cleaved by washing the decellularized tissue with PBS two times, soaking the tissue for 24 hours in green bean alpha-galactosidase dissolved in PBS at 100 units/L (75-200 units/L) at 2-6°C on a rotator. After 24 hours the tissue is rinsed with PBS two times and moved into PBS and antibiotics for storage.

### Example 2 - Recellularization

Stem cell culturing. Two methods were used in the collection of stem cells from patients. The first was a technique in which sternal blood is collected from patients who have undergone a sternotomy for cardiac surgery. After a sternotomy is performed, 0.1-0.5mL of sternal blood is aspirated and immediately taken for processing. A 75mL T25 plastic cell culture plate is filled with 15mL of cell culture media at 37°C. The blood is then placed directly into the media. The flask is then placed in an incubator at 37°C with 5.0% CO₂. The next day the media is aspirated, and the plate is washed with 15mL of PBS. The PBS is then aspirated and 15mL of media are added to the flask. The media is then changed every 3 days to allow for optimal growth conditions. Once the flask has reached 100% confluence, the cells can be removed from the flask and split between multiple flasks to allow for the growth of greater numbers.

The second technique was the collection of blood from the iliac crest. An iliac puncture is performed (a surgical procedure is not required) and bone marrow is aspirated. The blood is then separated into its components using Ficoll^{™} solution. The blood is diluted with an equal amount of Dulbecco's Modified Eagle Medium: Nutrient Mixture F-12 (DMEM) culture media. 20mL of the blood and culture media mixture is then layered on top of 15mL of Ficoll^{™} ensuring no mixing. The samples are then placed in a centrifuge and spun for 40 minutes. Once completed, the buffy coat is removed and directly plated as described above with the sternal whole blood. The media change intervals and protocols are the same as described above.

Once a sufficient number of cells have been cultured, they are removed from the culture plate. Before recellularization, the decellularized valve or valve material is moved to a culture media for 24 hours. The confluent plates are removed from the incubator and are washed with enough PBS to cover the plate. The PBS is then removed. TrypLE^{™} 10x is added to each plate with 1-3mm covering the entire plate to lift the adhered mesenchymal stem cells from the plate. The inventors expect that TrypLE^{™} 1x to 10x may be used. The plates are then moved back into the incubator and kept until the cells are confirmed to have lifted off of the plate with light microscopy. Once the cells have lifted, an equal amount of DMEM culture media is added to the plate. The solution containing the cells is collected and placed into a 50mL conical tube. The tubes are then centrifuged at 300-500g for 10-20 minutes at 2-8°C. Once centrifugation is complete, the cells should be in a visible pellet at the base of the tube. The solutions are then aspirated as close to the pellet as is possible without disturbing the cells. The cells are then washed with 10-25mL of PBS to remove any remaining TrpyLE^{™} and DMEM, inverted to mix and break up any pellets, and then centrifuged again at the same setting as above. After the centrifugation is complete, the solution is aspirated down to the pellet once again. The cells are then resuspended in DMEM culture media (by known methods that vary based on the number of cells and size of tissue being recellularized). The tissue is then incubated with the media and cells for 8-16 hours on a rotator at 37°C. After recellularization, the tissue can be used immediately or fixed using a glutaraldehyde solution.

During recellularization the inventors incubated the decellularized tissue scaffold in approximately 20 million cells in 40mL (approximately 500,000 cells/mL) of DMEM F12 for 12 hours. The inventors expect that incubation times between 8-16 hours, or 10-14 hours, will provide coverage of the decellularized tissue scaffold sufficient to provide immunocompatibility. The incubation period length is chosen to optimize coverage of the decellularized tissue scaffold. After 12 hours without any change to the media, the inventors found that the cells begin to die off. The inventors expect that increased cell concentrations will be required for longer and shorter incubations. The inventors expect that for an 8-hour incubation, a cell concentration in the range of 25-30 million cells in 40 mL of DMEM F12, or 625,000-750,000 cells/mL may be required. The inventors expect that for a 16-hour incubation, cells may begin to die, and so concentrations in the range of 20-25 million cells, or 500,000-625,000 cells/mL may be required to account for cell death. The inventors found 12 hours to be a preferable length of incubation to provide maximal coverage.

Several proofs of concept experiments have been conducted by the inventors and more are in the process of being performed. Native, decellularized, recellularized tissues have been exposed to blood in human in vitro, and rat in vivo models. The results of these experiments have shown a significant reduction in pro-inflammatory cytokines (immune system effectors) with the decellularized tissue matrix, and a further significant reduction with the recellularized scaffolds. Based on prior research and the research available in the literature the inventors expect that human pericardium and porcine aortic leaflets will demonstrate similar results.

### Example 3 - Bovine Pericardium

Bovine pericardium underwent the processes of decellularization and recellularization. The 20mL of collected whole blood collected from each patient was mixed with DMEM F12 media at equal parts and 10mL of the mixture was used to expose 3x3 mm of the native bovine pericardium (NBP) (n=6), decellularized bovine pericardium (DBP) (n=6), recellularized bovine pericardium (RBP) (n=6), and human pericardium (HP) (n=5). All exposure groups in 15mL tubes were then placed onto a rotator inside of a 37°C incubator. After 1 and 3 days, 2mL of the whole blood from the tube was taken out, underwent Ficoll-Paque^{™} gradient centrifugation (GE Healthcare, Sweden) and the top plasma layer was stored in an Eppendorf^{™} tube. They were flash-frozen with liquid nitrogen and stored in a -80°C freezer until the enzyme-linked immunosorbent assay (ELISA) analysis.

Proliferation Assay. Peripheral blood mononuclear cells (PBMC) from 10mL of whole blood were isolated by Ficoll-Paque^{™} gradient centrifugation (GE Healthcare, Sweden). The mononuclear cells were counted using Trypan Blue exclusion dye and were resuspended in an appropriate amount of "Complete Medium" comprised of RPMI 1640 medium containing 10% human serum, 2mM L-glutamine, 50 IU penicillin/mL, and 50 mL streptomycin/mL (Thermo Fisher Scientific, California) to create a solution containing 1 × 106 cells/mLA 0.5µM solution of CellTrace^{™} Violet (ThermoFisher, California) stain was made. Peripheral blood mononuclear cells (PMBCs) at a concentration of 1x 106 cells/mL were then incubated in the 0.5µM CellTrace^{™} Violet stain solution for 20 min. Unbound dye was absorbed by the addition of 5 times the volume of RPMI 1640 and incubated for 5 min. Cells were then centrifuged and resuspended in the complete medium. Aliquots of stained cells were then distributed into four 15mL tubes with native, decellularized, recellularized, and human pericardial tissue. After 5-days of culture, cells were stained with fluorescent antibodies against CD4, CD8, and CD3. Analysis of proliferation was performed using flow cytometry with a 405 nm excitation and a 450/40 bandpass emission filter. Proliferation was identified by a reduction in CellTrace^{™} Violet signal intensity, with each peak representing a duplication in cell number.

Qualitative Analysis. Native, decellularized, and human pericardial tissue composition and immune cell infiltration were analyzed qualitatively using histology, immunohistochemistry, and transmission and scanning electron microscopy. First, 1×1mm pieces of native and decellularized pericardial tissues were stained with DAPI to check for the presence or absence of bovine cells prior to the whole blood exposure. After 5 days of exposure with whole blood, pericardial tissues of all groups were cut into 5x5mm pieces and fixed in 4% paraformaldehyde. After 24 hours, the samples were transferred to 70% ethanol. The tissue samples were sectioned and underwent H&E staining, immunohistochemistry staining with anti-vimentin (interstitial cells), and immunofluorescence staining with anti-CD3 (T cells) and anti-CD68 (Macrophages). Finally, non-exposed representative samples of native and decellularized tissues were sectioned both in parallel and perpendicular orientations to the tissue and were analyzed using TEM and SEM.

Measurement of Cytokines. The supernatant containing cytokines that were stored in a -80°C freezer was thawed and underwent enzyme-linked immunosorbent assay (ELISA) analysis according to the manufacturer's recommended protocol for human TNF-α DuoSet^{™} ELISA Kit (Cat. No. DY210-05) and Human IFN-γ DuoSet ELISA kit (Cat. No. DY285B-05) (R&D Systems, Minneapolis). Absorbance reading at 450 nm and 540 nm were performed using Synergy^{™} H4 hybrid reader (Biotek, Winooski, Vermont).

Histology and Microscopy. NBP stained in a 4',6-diamidino-2-phenylindole (DAPI) stain showed extensive visible nuclear material. In contrast, DBP showed a complete absence of nuclear material within the region of the extracellular matrix in the background. After recellularization, DAPI stain again showed extensive coverage of the extracellular matrix by the mesenchymal cells. SEM imaging of native, decellularized and recellularized tissues showed comparable collagen morphology and distribution without any significant collagen disruption, fibrin deposition, and red blood cell trapping after the decellularization process. Cellular structures were found native and recellularized tissues whereas no cells were found on the decellularized tissue images. TEM visualizations of the extracellular matrix of native tissue, decellularized and recellularized tissue revealed clearance of bovine cells on decellularized tissue as well as the presence of mesenchymal cell structures seen on recellularized tissue. Extracellular matrix as indicated by the collagen banding on TEM were continually maintained throughout the tissue engineering process adding to the evidence that the process did not disturb the ultracellular structure.

Anti-vimentin stained NBP showed staining of interstitial cells found in all animal cells on the edges of the tissue whereas DBP showed the absence of interstitial cell staining at comparative sites confirming the effectiveness of the decellularization process. The recellularized tissue showed prominent vimentin staining, which is due to the protein being expressed by mesenchymal cells further adding to the evidence that the mesenchymal cells seated well on the decellularized xenograft.

Haematoxylin-eosin staining of native, decellularized, and recellularized tissues after 5 days of exposure depicted comparable overall morphology of extracellular matrix highlighted by preserved laminar architecture after the tissue engineering procedure. Similar patterns of extracellular matrix conservation were seen in Elastic Von Geison and Gomori's trichrome staining of the tissues where it demonstrated the presence of collagen in tissues that underwent the tissue engineering process. Taken together, histological and microscopic examination of tissues confirms the efficacy of the detergent-based decellularization process.

Immune Cell Infiltration. Hematoxylin and eosin stained section of native tissue showed innate immune cell infiltration with the presence of neutrophils and plasma cells whereas decellularized and recellularized tissue demonstrated no immune cell infiltrates. Immune cell infiltration was further characterized by immunofluorescence of native tissue that was sectioned after 5 days of exposure with human whole blood. T cell infiltrates (CD3) and macrophages (CD68) were depicted in the native tissue. This demonstrates that native tissue showed an early xenogenic innate and cell mediate immune response.

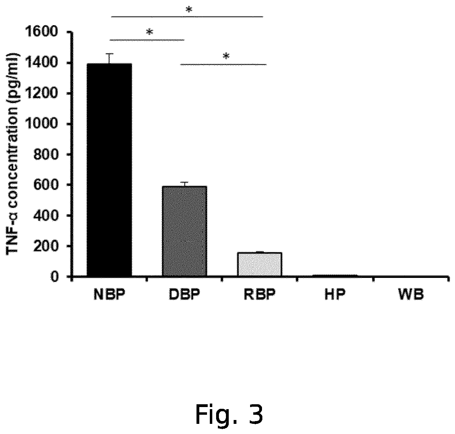

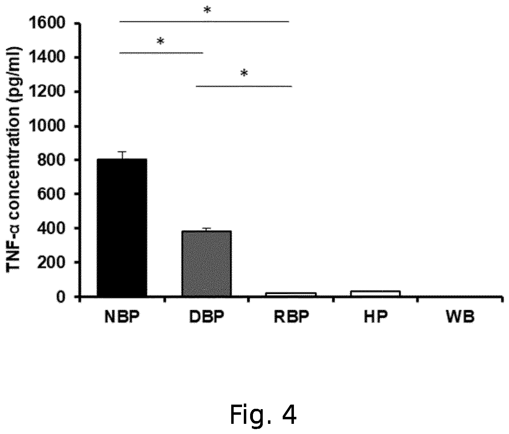

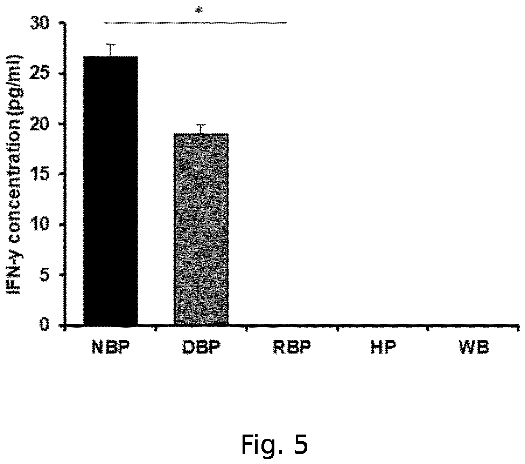

Proinflammatory Cytokines. As shown in Fig. 3, an ELISA analysis of TNF-α proinflammatory cytokine expression at day 1 of exposure demonstrated a significant decrease in cytokine production in the DBP exposure group in comparison to the NBP group (589.3 pg/mL vs 1388.5 pg/mL, p<0.02). Further, recellularization significantly decreased the cytokine expression in comparison to the DBP group (159.0 pg/mL vs 589.3 pg/mL, p<0.02). RBP exposure group cytokine level in comparison to HP showed no significant difference (159 pg/mL vs 9.4 pg/mL P>0.05). As shown in Fig. 4, day 3 of exposure also demonstrates a significant decrease in the cytokine production in the DBP exposure group in comparison to the NBP group (381.0 pg/mL vs 807.8 pg/mL, p<0.01). Recellularization again significantly decreased the cytokine expression in comparison to the DBP group (24.3 pg/mL vs 381.0 pg/mL, p<0.05). Once again, the RBP exposure group cytokine level in comparison to HP showed no significant difference (24.3 pg/mL vs 36.1 pg/mL P>0.05). To note, there was a decrease in the overall cytokine production for all exposure groups when comparing cytokine levels at day 1 and day 3, however, the trend of a significant decrease in cytokine levels from NBP to DBP to RBP was still present at both time points. IFN-γ proinflammatory cytokine expression at day 1 of exposure displayed no cytokine production. However, as shown in Fig. 5, at day 3 of exposure, the RBP exposure group cytokine level in comparison to the NBP group showed a significant decrease in the cytokine production (0 pg/mL vs 26.5 pg/mL P<0.05).

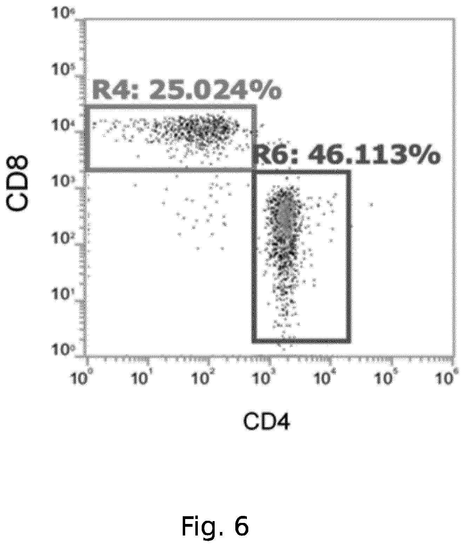

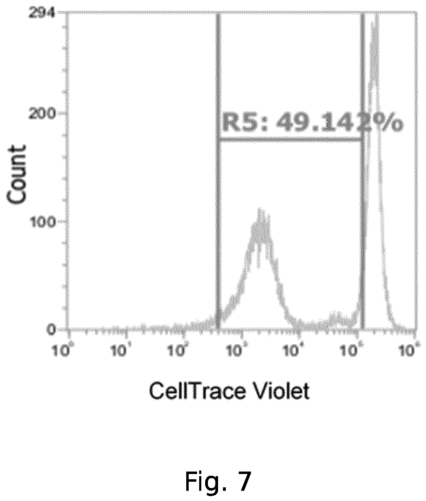

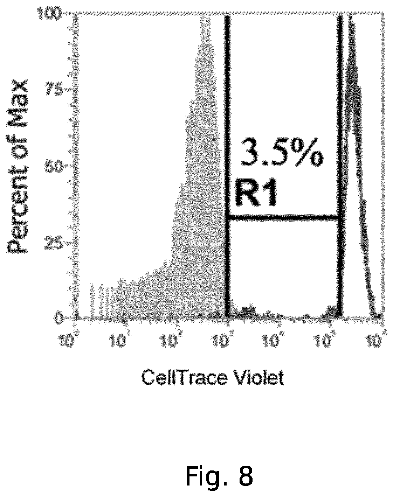

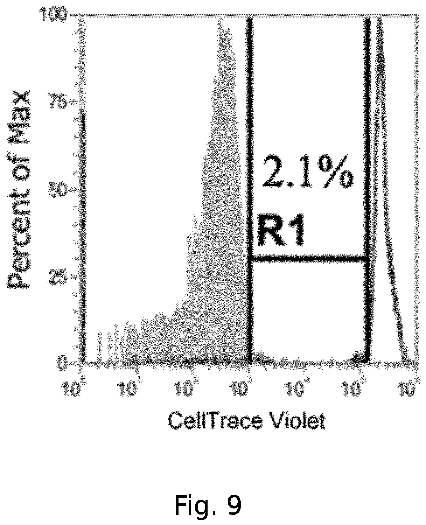

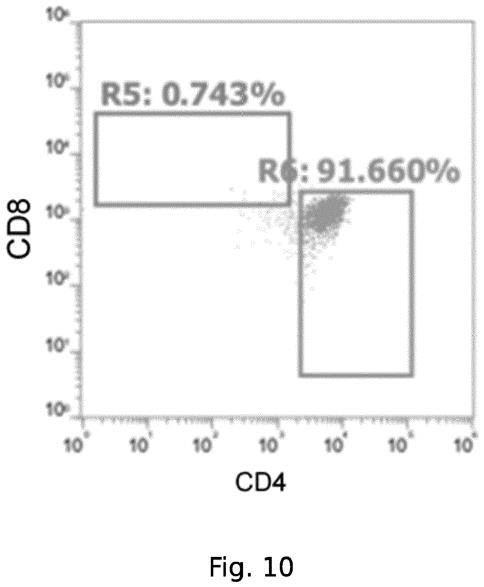

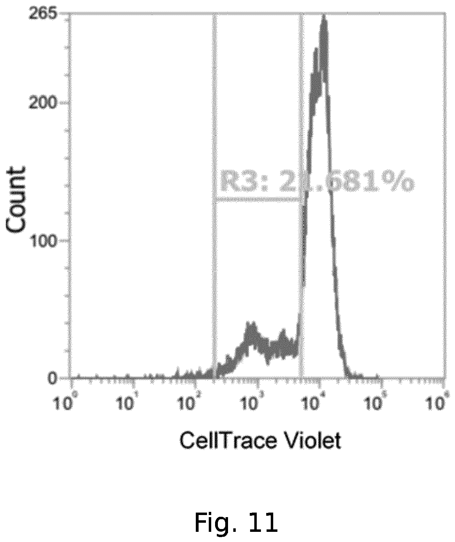

Flow Cytometry. Figs. 6 to 11 show the results of flow cytometry for NBP (Figs. 6 and 7), DBP (Fig. 8), HP (Fig. 9) and RBP (Fig. 10 and 11). CD 4+ T cell proliferation assay under flow cytometry for RBP shows significantly reduced T cell proliferation compared to the NBP group (49.1% vs 21.6%), where it displays increased proliferation of T cells indicated by peaks with fold fractional decrease corresponding to each proliferation of T cells. When compared to HP, the RBP group still demonstrated T cell proliferation (21.6% vs 2.1%).

### Example 4 - Porcine Extracellular Matrix

Patient Population. Patients undergoing elective cardiac surgery were consented preoperatively. Intraoperatively, pericardium, 20mL of heparinized whole blood, and samples of sternal bone marrow were collected.

Decellularization. A commercially available porcine extracellular matrix (Cormatrix^{™}, Georgia, USA) was used as the scaffold. Initially, 2-inch x 2-inch pieces of matrix were decellularized. The tissue was first washed with 25mL of phosphate-buffered saline (PBS) and then washed with 1% sodium dodecyl sulfate (SDS) (Bio-Rad Laboratories, California, USA) while oscillating at 40rpm at room temperature. The SDS was changed every hour for 4 changes in total. The fourth addition of SDS was left for 20 hours. The next day the SDS was removed and 25mL of Triton^{™} X-100 (Thermo Fisher Scientific, Massachusetts, USA) was added while oscillating at 40rpm at room temperature. After 1 hour the Triton^{™} X-100 was removed and replaced. This was repeated for a total of 4 changes, and after the left change was left for 20 hours. The next morning the Triton^{™} X-100 was removed and the tissue was rinsed with PBS and double distilled water (DDH₂O) until bubbles no longer formed. The tissue was then washed with 1% sodium chloride for 1 hour while oscillating at 40rpm at room temperature. The sodium chloride was removed, the tissue was again rinsed with PBS and DDH₂O. After rinsing 25mL of DNase and MgCl₂ was added and the tissue oscillated at 40rpm at 37°C. After aspirating, the tissues were rinsed twice with DDH₂O, and 25mL of 1% PBS penicillin-streptomycin mixture was added, the tissue was left oscillating at 40rpm at 37°C for 30 minutes. Once decellularization was complete the tissue was stored in 1% Hank's balanced salt solution with primocin (InvivoGen, California, USA) in a 4°C fridge until use.

Cell Culturing. Mesenchymal stem cells (MSCs) were collected from patients undergoing elective cardiac surgery at the Mazankowski Alberta Heart Institute, University of Alberta. A sample of blood from the sternal incision was collected and directly plated onto a T75 plastic cell culture flask (Thermo Fisher Scientific, Massachusetts, USA). The cells were incubated at 37°C with 5% CO₂ in DMEM F12 (Thermo Fisher Scientific, Massachusetts, USA) culture media completed with 20% fetal bovine serum, 0.2% ascorbic acid, and 0.2% primocin in order to only culture plastic adherent MSCs. The cells were washed with PBS and the media changed at 1 day after initial plating. The media was then changed every 3 days afterward and monitored regularly until the flask had reached 100% confluence at which point they contained approximately 1 million cells. 15mL of TrypLE^{™} Select Enzyme 10x, no phenol red (Thermo Fisher Scientific, Massachusetts, USA) was added and incubated for 30 minutes to trypsinize the cells. 15mL of complete DMEM F12 was then added and the cells and solution were transferred to a 50mL conical tube for centrifugation at 400g, 18°C for 15min. The solution was aspirated, the cells were resuspended in PBS and underwent 1 additional cycle of centrifugation. The cell pellet was then resuspended in DMEM F12 media and either plated onto multiple flasks until it reached 95% confluency or used immediately for recellularization.

Recellularization. Recellularization began by incubating a 3mm by 3mm piece of tissue in 10mL of DMEM F12 culture media on a rotator at 37°C for 24h. The next day, approximately 1 million MSCs isolated as noted above would be resuspended in 4mL of DMEM F12 culture media. The tissue was then placed into the media in a 5mL centrifuge tube (MTC Bio, New Jersey, USA) including stem cells, and was placed on a rotator at 37°C for 12h.

Exposure. The 20mL of blood collected would be mixed with 20mL of DMEM F12 culture media. 10mL of whole blood was then moved into individual 15mL polypropylene centrifuge tubes (Thermo Fisher Scientific, Massachusetts, USA) and native porcine matrix (n=9), decellularized porcine matrix (n=9), recellularized porcine matrix (n=6), and autologous human pericardium (n=9) were placed into individual tubes. The tubes were then placed onto a rotator and incubated at 37°C. At days 1, 3, and 5 after exposure, 2 mL of whole blood was collected from each tube and placed into a 2mL Eppendorf^{™} tube (Thermo Fisher Scientific, Massachusetts, USA). The samples were then centrifuged at 400g, 18°C for 15min. The serum was collected and move into a new tube, the pellet was discarded. The tubes containing the serum were flash-frozen in liquid nitrogen and stored at - 80°C until enzyme-linked immunosorbent assay (ELISA) analysis.

Qualitative Analysis. Confirmation of successful decellularization and recellularization was completed by 4',6-diamidino-2-phenylindole (DAPI) staining. Tissue samples at each stage of processing including native, decellularized, and recellularized samples were stained with DAPI in order to identify the presence of original cells, the absence of those cells after decellularization, and the presence of MSCs after recellularization.

Quantitative Analysis. The serum containing cytokines that were stored at - 80°C was thawed and underwent ELISA analysis according to the manufacturer's recommended protocol for human TNF-α DuoSet^{™} ELISA Kit (Cat. No. DY210-05) and Human IL1β DuoSet^{™} ELISA kit (Cat. No. DY201-05) (R&D Systems, Minneapolis). Absorbance reading at 450 nm and 540 nm were performed using Synergy^{™} H4 hybrid reader (Biotek, Winooski, Vermont).

Statistical Analysis. A comparison of the data among the groups was analyzed using unpaired t-test, and statistical significance was taken at p<0.05. Comparison of variation between groups was conducted using a one way ANOVA, statistical significance was set at p<0.05.

Qualitative Analysis. Native matrix (NCM) stained with DAPI displays the presence of nuclear material. In contrast, the DCM showed the complete absence of nuclear material on DAPI staining. After recellularization, the DAPI stain showed the extensive presence of nuclear material from the MSCs used to recellularize the decellularized matrix (DCM) forming a recellularized matrix (RCM)

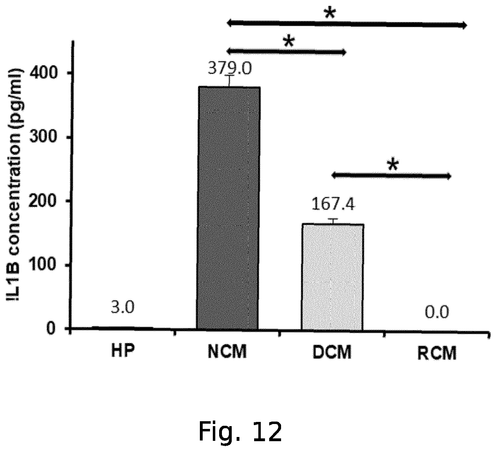

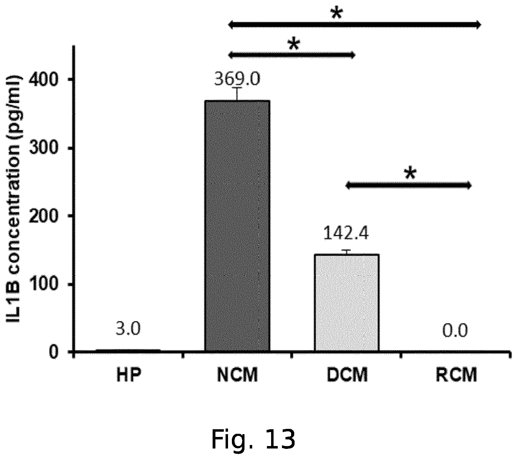

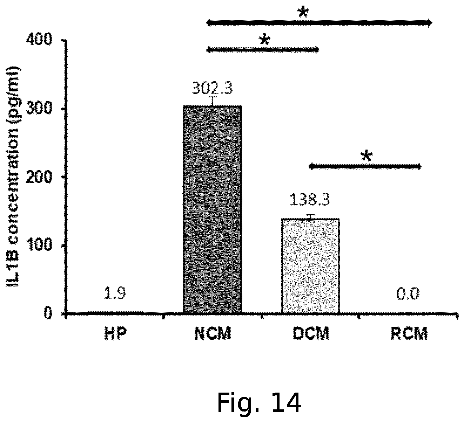

ELISA. ELISA of IL1β demonstrated a significant reduction in cytokine production in the DCM group when compared to the NCM group on day 1 (167.4pg/mL vs 379.0pg/mL, p=0.006) as shown in Fig. 12, day 3 (142.4pg/mL vs 369.0pg/mL, p=0.007) as shown in Fig. 13 and day 5 (138.3pg/mL vs 302.3pg/mL, p=0.02) as shown in Fig. 14. In Fig. 12, NCM: Native matrix, DCM: Decellularized matrix, RCM: Recellularized matrix, HP: Autologous Human Pericardium. Unpaired T-Test NCM:DCM P=0.006*, NCM:RCM P<0.001*, DCM:RCM P=0.001*. In Fig. 13, Unpaired T-Test NCM:DCM P=0.007*, NCM:RCM P<0.001*, DCM:RCM P=0.003*. In Fig. 14, Unpaired T-Test NCM:DCM P=0.002*, NCM:RCM P<0.001*, DCM:RCM P<0.001 *.

Similarly, a significant reduction was demonstrated from the recellularized to the native tissue at day 1 (O.Opg/mL vs 379.0pg/mL, p<0.001), day 3 (O.Opg/mL vs 369.0pg/mL, p<0.001) and day 5 (O.Opg/mL vs 83.0pg/mL, p=0.03). A significant reduction in IL1β production was also noted from the RCM when compared to the DCM at day 1 (0.0pg/mL vs 167.4pg/mL, p=0.001) as shown in Fig. 12, day 3 (O.Opg/mL vs 142.4pg/mL, p=0.003) as shown in Fig. 13 and day 5 (O.Opg/mL vs 138.3pg/mL, p<0.001) as shown in Fig. 14. ANOVA demonstrated significant variation between group at day 1 F(2, 21)= 14.6, p<0.001, at day 3 F(2, 21)= 12.1, p<0.001 and day 5 F(2, 21)= 10.0, p<0.001.

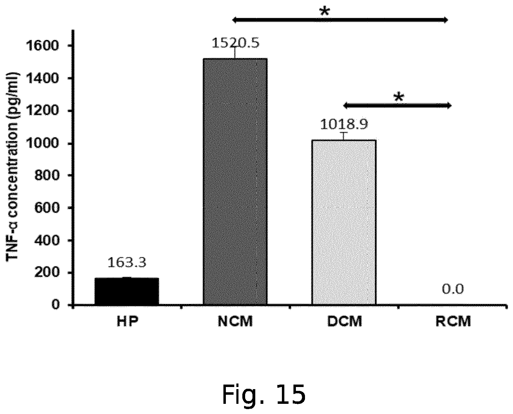

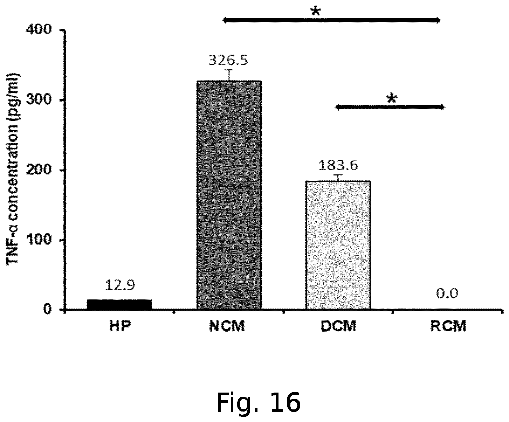

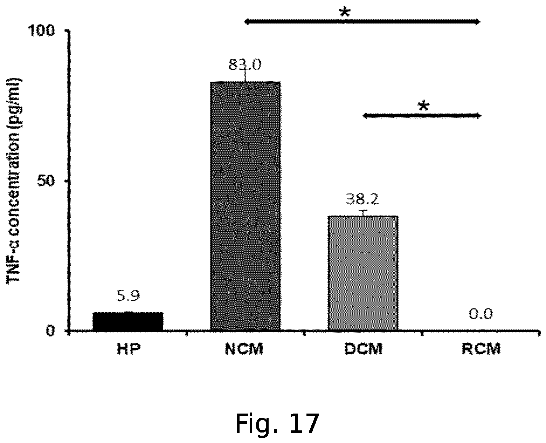

ELISA of TNFα demonstrated similar results to the previous ELISA, although there was no significant reduction from the native matrix to the decellularized matrix. A significant reduction was found from recellularized matrix when compared to the native matrix on day 1 (0.0pg/mL vs 1520.5pg/mL, p<0.001) as shown in Fig. 15, day 3 (O.Opg/mL vs 326.5pg/mL, p=0.004) as shown in Fig. 16 and day 5 (O.Opg/mL vs 83.0pg/mL, p<0.001) as shown in Fig. 17. In Fig. 15, Unpaired T-Test NCM:DCM P=0.079, NCM:RCM P<0.001*, DCM:RCM P=0.003*. In Fig. 16, Unpaired T-Test NCM:DCM P=0.12, NCM:RCM P=0.004*, DCM:RCM P=0.002*. In Fig. 17, Unpaired T-Test NCM:DCM P=0.18, NCM:RCM P=0.046*, DCM:RCM P=0.03*.

Finally, a significant reduction in TNFα production was noted from the RCM to the DCM at day 1 (0.0pg/mL vs 1018.9pg/mL, p=0.003) as shown in Fig. 15, day 3 (O.Opg/mL vs 183.6pg/mL, p0.002) as shown in Fig. 16 and day 5 (O.Opg/mL vs 38.2pg/mL, p=0.03) as shown in Fig. 17. ANOVA demonstrated significant variation between group at day 1 F(2, 21)= 10.8, p<0.001 and at day 3 F(2, 21)= 3.94, p= 0.04. Variation between groups was not significant at day 5.

### Example 5 - Guinea Pig Valves

Decellularization. Explanted aortic valve constructs from female Hartley guinea pigs were decellularized using the same method described in Example 4.

Cell Culturing. Syngeneic Sprague-Dawley rat mesenchymal stem cells (MSCs) were used for recellularization. Rat MSCs were plated, cultured, and used for recellularization as described above in Example 4.

Recellularization. Recellularization began by incubating an individual aortic valve construct 10mL of DMEM F12 culture media on a rotator at 37°C for 24h. The next day, approximately 1 million rat MSCs isolated as noted above would be resuspended in 4mL of DMEM F12 culture media. The tissue was then placed into the media in a 5mL centrifuge tube (MTC Bio, New Jersey, USA) including stem cells, and was placed on a rotator at 37°C for 12h. The recellularized tissue was then ready for qualitative and quantitative analysis.

Exposure. Explanted aortic valve constructs from female Hartley guinea pigs were procured and decellularized, followed by recellularization with syngeneic Sprague-Dawley rat MSCs. The recellularized aortic valve xenografts were then implanted into the infrarenal aorta of recipient female Sprague-Dawley rats. Grafts were implanted as either syngeneic grafts, non-decellularized (fresh), decellularized, and recellularized xenografts. Grafts were sewn end-to-end with continuous 10-0 nylon suture. Rats were euthanized after 7-days, exsanguinated, and the grafts explanted. Total serum immunoglobulin was quantified and histological analysis was performed to analyze the immune response

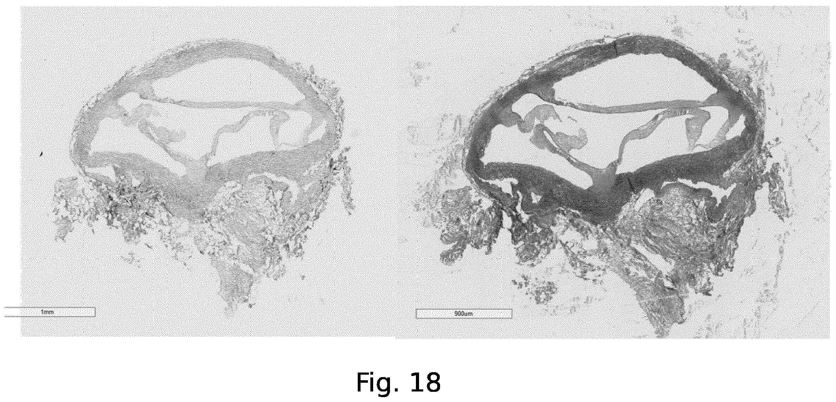

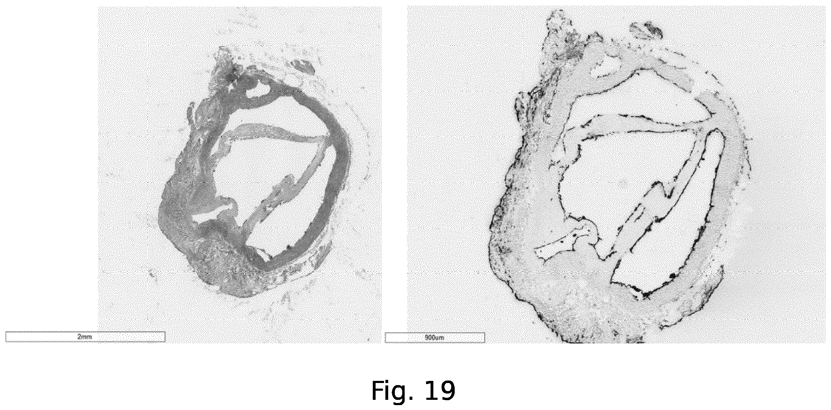

Qualitative Analysis. Confirmation of successful decellularization and recellularization was completed by 4',6-diamidino-2-phenylindole (DAPI) staining. Tissue samples at each stage of processing including native, decellularized, and recellularized samples were stained with DAPI in order to identify the presence of original cells, the absence of those cells after decellularization, and the presence of MSCs after recellularization. Gross histology was performed with hemotoxylin and eosin (H&E) and vimentin staining, confirming the presence of cellular material in the native xenograft. In Fig. 18, H&E and vimentin staining of the decellularized xenograft confirmed that the decellularization procedure did not adversely affect the gross structure of the valve while removing all cellular material. In Fig. 19, H&E and vimentin staining of the recellularized xenograft confirmed that a confluent layer of cells had adhered to the valve surface.

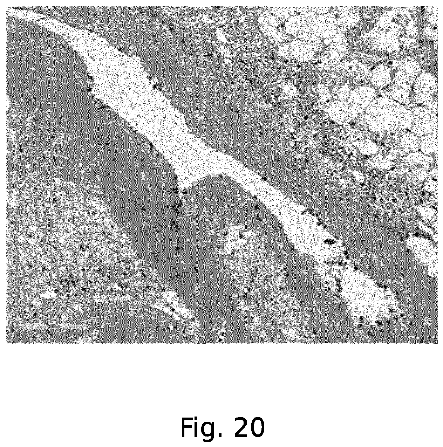

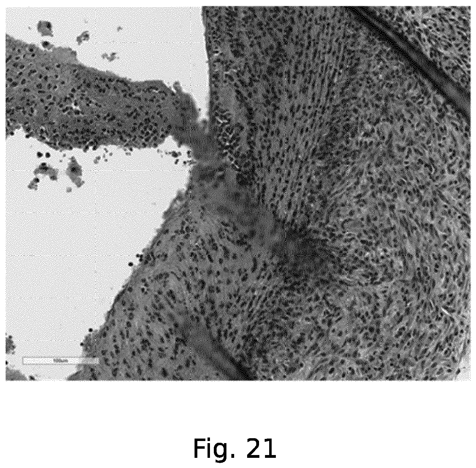

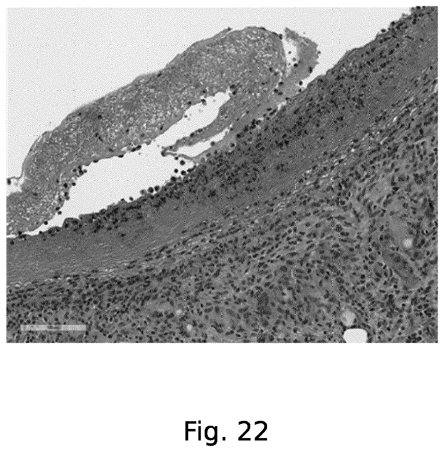

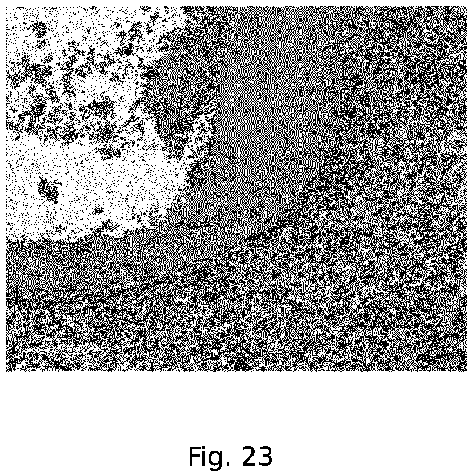

As shown in Figs. 20-23, H&E staining demonstrated reduced cellular infiltrates in the recellularized guinea pig xenograft when compared to the fresh and decellularized grafts.

Quantitative Analysis. The serum containing cytokines that were stored at - 80°C was thawed and underwent ELISA analysis according to the manufacturer's recommended protocol for rat IgG (Cat No. E111-128) (Bethyl Laboratories Inc. Montgomery, Texas, USA). Absorbance reading at 450 nm and 540 nm were performed using Synergy^{™} H4 hybrid reader (Biotek, Winooski, Vermont).

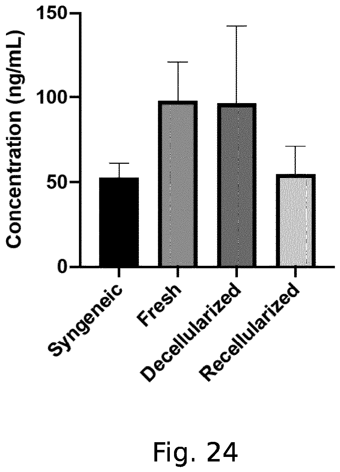

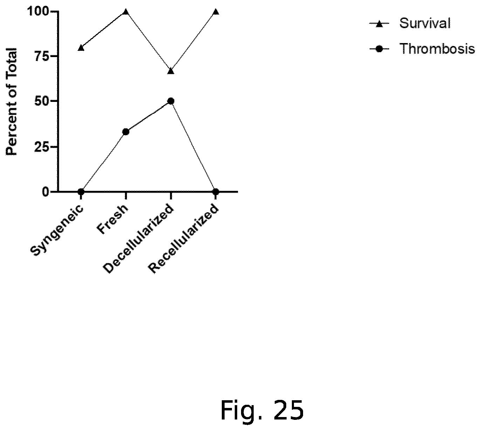

Fig. 24 shows total serum immunoglobulin G at 7 days post-implant. Fig. 25 shows survival and thrombosis of implanted grafts at 7 days post-implant.

The inventors found similar rates of survival between the various implant types, although decellularized grafts had a much higher incidence of graft thrombosis.

### Example 6 - Pig Aortic Leaflet with or without Alpha-Galactose Cleavage

The inventors have demonstrated an effective method in cleavage of alpha-galactose from a porcine aortic leaflet. Alpha-galactose is a carbohydrate found on most mammalian tissue although is not found in primates. This carbohydrate has been hypothesized to contribute to the robust immune response to xenogenic implants into humans.

Alpha-galactose cleavage. After tissue decellularization, the tissue was then be moved into a new tube containing 15mL of 100units/L alpha-galactosidase in PBS. The tissue is placed on a rotator for 24 hours at 4°C.

Based on previous experiments and results in the literature, the inventors expect that cleavage of alpha-galactose will further reduce the immune response and will demonstrate reductions in proinflammatory cytokine production in in-vitro models.

### Example 7 - Human Pericardium

Patient Population. Patients undergoing elective cardiac surgery were consented preoperatively. Intraoperatively, pericardium, 20mL of heparinized whole blood, and samples of sternal bone marrow were collected.

Decellularization. Human pericardium was used as the scaffold. Initially, 5.08-cm (2-inch) × 5.08-cm (2-inch) pieces of human pericardium were decellularized as described above in Example 4.

Cell Culturing. Mesenchymal stem cells (MSCs) were collected and cultured as described above in Example 4.

Recellularization. Recellularization was performed as described above in Example 4.

*Exposure.* The exposure protocol was performed as described above in Example 4. Human pericardium taken from one patient would be decellularized and recellularized with another patient's MSCs. The recellularized matrix would then be exposed to peripheral blood mononuclear cells (PBMCs) collected from the same patient that the MSCs were collected from creating an autograft model.

Quantitative Analysis. The serum containing cytokines that were stored at - 80°C was thawed and underwent ELISA analysis according to the manufacturer's recommended protocol for human TNF-α DuoSet ELISA Kit (Cat. No. DY210-05) (R&D Systems, Minneapolis). Absorbance reading at 450 nm and 540 nm were performed using Synergy H4 hybrid reader (Biotek, Winooski, Vermont).

Statistical Analysis. A comparison of the data among the groups was analyzed using unpaired t-test, and statistical significance was taken at p<0.05.

*ELISA.* ELISA of TNFα did not demonstrate significant reductions from the native pericardium to the decellularized or recellularized human pericardium. At days 1 and 3 decellularized human pericardium produced a greater TNF-α concentration than native pericardium and autologous cell recellularized human pericardium reduced the TNF-α concentration to a level similar to the native pericardium.

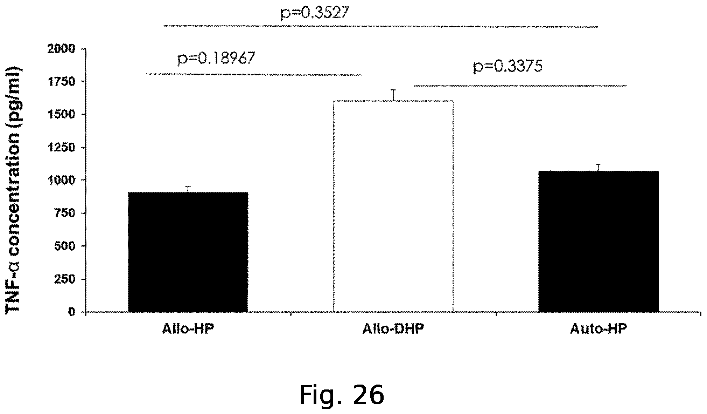

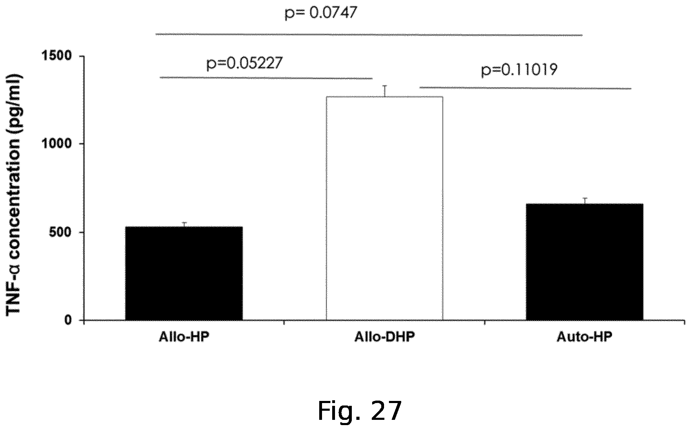

Fig. 26 shows a TNF-α cytokine concentration at 1-day post-exposure with PBMCs for one day. Fig. 27 shows a TNF-α cytokine concentration at 1-day post-exposure with PBMCs for three days.

In the claims, the word "comprising" is used in its inclusive sense and does not exclude other elements being present. The indefinite articles "a" and "an" before a claim feature do not exclude more than one of the feature being present.

## Claims

1. A method of forming an immunocompatible scaffold for a recipient, comprising contacting a decellularized tissue matrix in vitro with eukaryotic cells immunocompatible to the recipient to cover an exterior surface of the decellularized tissue matrix to form the immunocompatible scaffold, the decellularized tissue formed from tissue xenogenic to the recipient; and fixing the immunocompatible scaffold with a fixing agent.

2. The method of claim 1 further comprising contacting the decellularized tissue matrix with alpha-galactosidase prior to recellularizing the decellularized tissue matrix.

3. The method of any one of claims 1 or 2 in which the fixing agent is glutaraldehyde.

4. The method of any one of claims 1 to 3 in which the decellularized tissue matrix further comprises heart valve tissue.

5. The method of any one of claims 1 to 4 in which the recipient is human and the tissue porcine or bovine tissue.

6. The method of claim 1 further comprising decellularizing a tissue xenogenic to a recipient to form the decellularized tissue matrix, the method of decellularizing the tissue xenogenic to a recipient comprising:
immersing the tissue in an anionic detergent solution;
removing the anionic detergent solution from the tissue;
immersing the tissue in a nonionic detergent solution;
removing the nonionic detergent solution from the tissue;
rinsing the tissue with one or more rinsing solutions to form a decellularized tissue matrix; and
contacting the decellularized tissue matrix with alpha-galactosidase.

7. The method of claim 6 in which the one or more rinsing solutions collectively comprising a buffer solution, double distilled water, sodium chloride, a nuclease and antibiotics.

8. The method of any one of claims 6 to 7 in which the method is completed in less than 4 days.

9. The method of any one of claims 1 to 8 in which contacting a decellularized tissue matrix in vitro with eukaryotic cells immunocompatible to the recipient to cover an exterior surface of the decellularized tissue matrix further comprises contacting the decellularized tissue matrix with eukaryotic cells immunocompatible to the recipient to form a layer of eukaryotic immunocompatible cells covering at least 50% of the decellularized tissue matrix.

10. An immunocompatible scaffold comprising:
a decellularized xenogenic tissue covered with fixed eukaryotic cells immunocompatible to a recipient and forming a shelf-stable product.

11. The immunocompatible scaffold of claim 10 in which the eukaryotic cells cover at least 50% of an external surface of the decellularized tissue matrix.

12. The immunocompatible scaffold of claim 10 in which the eukaryotic cells cover at least 80% of an external surface of the decellularized tissue matrix.

13. The immunocompatible scaffold of any one of claims 10 to 12 in which the eukaryotic cells comprise cells homologous or autologous to the recipient.

14. The immunocompatible scaffold of any one of claims 10 to 13 in which the eukaryotic cells further comprise mesenchymal stem cells.

15. The immunocompatible scaffold of any one of claims 10 to 14 in which the decellularized xenogenic tissue further comprises xenogenic heart valve tissue.

## Patentansprüche

1. Verfahren zur Herstellung eines immunokompatiblen Gerüsts für einen Empfänger, umfassend Kontaktieren einer dezellularisierten Gewebematrix in vitro mit eukaryotischen Zellen, die immunokompatibel mit dem Empfänger sind, um eine äußere Oberfläche der dezellularisierten Gewebematrix zu bedecken, um das immunokompatible Gerüst herzustellen, wobei das dezellularisierte Gewebe aus Gewebe hergestellt ist, das xenogen für den Empfänger ist; und Fixieren des immunokompatiblen Gerüsts mit einem Fixiermittel.

2. Verfahren nach Anspruch 1, ferner umfassend Kontaktieren der dezellularisierten Gewebematrix mit Alpha-Galactosidase vor Rezellularisieren der dezellularisierten Gewebematrix.

3. Verfahren nach einem der Ansprüche 1 oder 2, wobei das Fixiermittel Glutaraldehyd ist.

4. Verfahren nach einem der Ansprüche 1 bis 3, wobei die dezellularisierte Gewebematrix ferner Herzklappengewebe umfasst.

5. Verfahren nach einem der Ansprüche 1 bis 4, wobei der Empfänger menschlich und das Gewebe Schweine- oder Rindergewebe ist.

6. Verfahren nach Anspruch 1, ferner umfassend Dezellularisieren eines Gewebes, das xenogen für einen Empfänger ist, um die dezellularisierte Gewebematrix herzustellen, wobei das Verfahren zum Dezellularisieren des Gewebes, das xenogen für einen Empfänger ist, Folgendes umfasst:
Eintauchen des Gewebes in eine anionische Reinigungslösung;
Entfernen der anionischen Reinigungslösung aus dem Gewebe;
Eintauchen des Gewebes in eine nichtionische Reinigungslösung;
Entfernen der nichtionischen Reinigungslösung aus dem Gewebe;
Spülen des Gewebes mit einer oder mehreren Spüllösungen, um eine dezellularisierte Gewebematrix herzustellen; und
Kontaktieren der dezellularisierten Gewebematrix mit Alpha-Galactosidase.

7. Verfahren nach Anspruch 6, wobei die eine oder mehreren Spüllösungen insgesamt eine Pufferlösung, doppelt destilliertes Wasser, Natriumchlorid, eine Nuklease und Antibiotika umfassen.

8. Verfahren nach einem der Ansprüche 6 bis 7, wobei das Verfahren in weniger als 4 Tagen abgeschlossen ist.

9. Verfahren nach einem der Ansprüche 1 bis 8, wobei das Kontaktieren einer dezellularisierten Gewebematrix in vitro mit eukaryotischen Zellen, die immunokompatibel mit dem Empfänger sind, um eine äußere Oberfläche der dezellularisierten Gewebematrix zu bedecken, ferner Kontaktieren der dezellularisierten Gewebematrix mit eukaryotischen Zellen umfasst, die immunokompatibel mit dem Empfänger sind, um eine Schicht aus eukaryotischen immunokompatiblen Zellen herzustellen, die zumindest 50% der dezellularisierten Gewebematrix bedeckt.

10. Immunokompatibles Gerüst, umfassend:
ein dezellularisiertes xenogenes Gewebe, das mit fixierten eukaryotischen Zellen bedeckt ist, die immunokompatibel mit einem Empfänger sind und ein lagerstabiles Produkt herstellen.

11. Immunokompatibles Gerüst nach Anspruch 10, wobei die eukaryotischen Zellen zumindest 50% einer äußeren Oberfläche der dezellularisierten Gewebematrix bedecken.

12. Immunokompatibles Gerüst nach Anspruch 10, wobei die eukaryotischen Zellen zumindest 80% einer äußeren Oberfläche der dezellularisierten Gewebematrix bedecken.

13. Immunokompatibles Gerüst nach einem der Ansprüche 10 bis 12, wobei die eukaryotischen Zellen Zellen umfassen, die homolog oder autolog zu dem Empfänger sind.

14. Immunokompatibles Gerüst nach einem der Ansprüche 10 bis 13, wobei die eukaryotischen Zellen ferner mesenchymale Stammzellen umfassen.

15. Immunokompatibles Gerüst nach einem der Ansprüche 10 bis 14, wobei das dezellularisierte xenogene Gewebe ferner xenogenes Herzklappengewebe umfasst.

## Revendications

1. Procédé de formation d'un échafaudage immunocompatible pour un receveur, comprenant la mise en contact d'une matrice de tissu décellularisé in vitro avec des cellules eucaryotes immunocompatibles avec le receveur de manière à recouvrir une surface externe de la matrice de tissu décellularisé afin de former l'échafaudage immunocompatible, le tissu décellularisé étant formé à partir de tissu xénogénique par rapport au receveur ; et la fixation de l'échafaudage immunocompatible avec un agent de fixation.

2. Procédé selon la revendication 1, comprenant en outre la mise en contact de la matrice de tissu décellularisé avec de l'alpha-galactosidase avant la recellularisation de la matrice de tissu décellularisé.

3. Procédé selon l'une quelconque des revendications 1 ou 2, ledit agent de fixation étant le glutaraldéhyde.

4. Procédé selon l'une quelconque des revendications 1 à 3, ladite matrice de tissu décellularisé comprenant en outre du tissu valvulaire cardiaque.

5. Procédé selon l'une quelconque des revendications 1 à 4, ledit receveur étant humain et ledit tissu un tissu porcin ou bovin.

6. Procédé selon la revendication 1, comprenant en outre la décellularisation d'un tissu xénogénique par rapport à un receveur de manière à former la matrice de tissu décellularisé, le procédé de décellularisation du tissu xénogénique par rapport à un receveur comprenant :
l'immersion du tissu dans une solution détergente anionique ;
le retrait de la solution détergente anionique du tissu ;
l'immersion du tissu dans une solution détergente non ionique ;
le retrait de la solution détergente non ionique du tissu ;
le rinçage du tissu avec une ou plusieurs solutions de rinçage de manière à former une matrice de tissu décellularisé ; et
la mise en contact de la matrice de tissu décellularisé avec de l'alpha-galactosidase.

7. Procédé selon la revendication 6, lesdites une ou plusieurs solutions de rinçage comprenant collectivement une solution tampon, de l'eau bidistillée, du chlorure de sodium, une nucléase et des antibiotiques.

8. Procédé selon l'une quelconque des revendications 6 à 7, ledit procédé étant réalisé en moins de 4 jours.

9. Procédé selon l'une quelconque des revendications 1 à 8, ladite mise en contact d'une matrice de tissu décellularisé in vitro avec des cellules eucaryotes immunocompatibles avec le receveur de manière à recouvrir une surface externe de la matrice de tissu décellularisé comprenant en outre la mise en contact de la matrice de tissu décellularisé avec des cellules eucaryotes immunocompatibles avec le receveur de manière à former une couche de cellules eucaryotes immunocompatibles recouvrant au moins 50 % de la matrice de tissu décellularisé.

10. Echafaudage immunocompatible comprenant :
un tissu xénogénique décellularisé recouvert de cellules eucaryotes fixes immunocompatibles avec un receveur et la formation d'un produit à longue durée de conservation.

11. Echafaudage immunocompatible selon la revendication 10, lesdites cellules eucaryotes recouvrant au moins 50 % d'une surface externe de la matrice de tissu décellularisé.

12. Echafaudage immunocompatible selon la revendication 10, lesdites cellules eucaryotes recouvrant au moins 80 % d'une surface externe de la matrice de tissu décellularisé.

13. Echafaudage immunocompatible selon l'une quelconque des revendications 10 à 12, lesdites cellules eucaryotes comprenant des cellules homologues ou autologues par rapport au receveur.

14. Echafaudage immunocompatible selon l'une quelconque des revendications 10 à 13, lesdites cellules eucaryotes comprenant en outre des cellules souches mésenchymateuses.

15. Echafaudage immunocompatible selon l'une quelconque des revendications 10 à 14, ledit tissu xénogénique décellularisé comprenant en outre du tissu valvulaire cardiaque xénogénique.
